# EUROPÄISCHE PATENTSCHRIFT

(11) **EP 0 842 431 B1**
(45) Veröffentlichungstag und Bekanntmachung des Hinweises auf die Patenterteilung: **24.09.2003**
(21) Anmeldenummer: 97924995.0
(22) Anmeldetag: 27.05.1997
(51) Int. Cl.: G01N 33/569

(54) **DIAGNOSTISCHES VERFAHREN ZUR ERKENNUNG SUBKLINISCH AN PARATUBERKULOSE ERKRANKTER SÄUGER**
DIAGNOSTIC PROCESS FOR RECOGNISING MAMMALS AFFECTED SUBCLINICALLY BY PARATUBERCULOSIS
METHODE DE DIAGNOSTIC POUR DEPISTER LA PARATUBERCULOSE SUBCLINIQUE CHEZ DES MAMMIFERES

(30) Priorität: 29.05.1996 DE 19621488
(43) Veröffentlichungstag der Anmeldung: 20.05.1998
(73) Patentinhaber: Gerlach, Gerald-F., 30449 Hannover (DE)
(72) Erfinder: Gerlach, Gerald-F., 30449 Hannover (DE)
(74) Vertreter: Minderop, Ralph H., Dr. rer. nat.
(86) Internationale Anmeldenummer: EP9702733
(87) Internationale Veröffentlichungsnummer: WO97045742

(56) Entgegenhaltungen:
- EP-A- 0 184 511
- WO-A-92/14151
- WO-A-92/16628
- WO-A-94/14069
- WO-A-97/26007
- US-A- 4 746 511
- SUGDEN ET AL: "serodiagnosis of ovine paratuberculosis, using lipoarabinomannan in an enzyme linked immunosorbent assay" AM.J.VET.RES, Bd. 50, Nr. 6, Juni 1989, Seiten 850-854, XP002041539 in der Anmeldung erwähnt
- SUGDEN ET AL: "Lipoarabinomannan and lipid free Arabinomannan Antigens of Mycobacterium paratuberculosis" INFECTION AND IMMUNITY, Bd. 55, Nr. 3, März 1987, Seiten 762-770, XP002041483 in der Anmeldung erwähnt
- U. Jark et al., Development of an ELISA technique for serodiagnosis of bovine paratuberculosis, Veterinary Microbiology, 1997, Vol. 51, pp. 189-198

## Beschreibung

Die Erfindung betrifft eine immunologische Testeinrichtung zum Nachweis der Paratuberkulose, ein Verfahren zur Herstellung einer solchen Testeinrichtung sowie ein immunologisches Verfahren zur Erkennung der Paratuberkulose.

Die Paratuberkulose von Säugern, insbesondere des Rindes, ist eine Krankheit mit weltweitem Vorkommen und großer wirtschaftlicher Bedeutung. Die Paratuberkulose des Rindes ist nicht heilbar und in Deutschland meldepflichtig. Die Erkrankung hat eine Inkubationszeit von zwei oder mehreren Jahren, innerhalb welcher infizierte Tiere den Erreger, *Mycobacterium paratuberculosis,* intermittierend mit Kot und Milch ausscheiden. Während die Diagnosestellung an klinisch erkrankten Tieren aufgrund klinischer und mikrobiologischer Parameter relativ leicht möglich ist, ist die Erkrankung latent erkrankter Tiere - also potentieller Ausscheider - und damit auch die Sanierung mit Paratuberkulose infizierter Bestände bisher nicht zuverlässig möglich.

Mikroskopische Verfahren haben nur zur Bestätigung der Diagnose bei klinisch kranken Tieren eine Bedeutung, da eine Erregerzahl von größer 10⁴/g Kot für den Nachweis erforderlich ist.

Kulturelle diagnostische Verfahren werden durch das langsame Wachstum des Erregers erschwert, so daß die Untersuchungsdauer länger als 8 Wochen beträgt. Zudem ist das Ergebnis der kulturellen Untersuchung nur bei positivem Ausfall aussagekräftig, da aufgrund der intermittierenden Erregerausscheidung die sichere Erkennung von *M. paratuberculosis* negativer Tiere nicht möglich ist.

Die serologische Untersuchung mit einem ELISA scheint das derzeit beste zur Verfügung stehende Diagnoseverfahren zu sein. Allerdings ist die Sensitivität der von der United States Department Of Agriculture (USDA) zugelassenen diagnostischen Tests nicht optimal. Das liegt möglicherweise daran, daß das Antigen von einem *M.* paratuberculosis Stamm gewonnen wird, der inzwischen als *M. avium* reklassifiziert wurde. Es handelt sich somit um ein heterologes Antigen. Andere Tests (Vannuffel et al. 1994) sind bisher nicht zugelassen.

Die Bereitstellung zuverlässiger immunologischer Nachweisverfahren scheiterte bisher auch daran, daß sich Polysaccharid- und Lipopolysaccharid-Antigene, wie sie zum Beispiel von Sugden et al. Infection and Imunity, März 1987, 762 - 770 beschrieben wurden, von *M. paratubercolosis* nicht effizient präpariert und an ELISA-Platten nicht in ausreichendem Umfang adsorbiert werden konnten.

Sugden et al. beschreiben in J. Clin. Microbiol., 1991, Bd. 29, Heft 8, 1659 - 1664 sowie in Am. J. Vet. Res., 1989, Bd. 50, Heft 6, 850 - 854 die Reinigung und Charakterisierung der Antigene A, D und LAM aus *M. Paratuberculosis,* sowie deren Verwendung in Diagnoseverfahren zur Erkennung von Paratuberkulose bei Schafen. Als Diagnoseverfahren wurde das ELISA Verfahren angewendet, wobei die isolierten Antigene auf Kunststoff-Mikrotiterplatten (z.B. aus Polystyrol) aufgetragen wurden.

Der Erfindung liegt nun die Aufgabe zugrunde, eine immunologische Testeinrichtung sowie ein Diagnoseverfahren zum sicheren Nachweis einer Paratuberkulose-Erkrankung bereit zu stellen.

Gelöst wird diese Aufgabe durch eine immunologische Testeinrichtung zum Nachweis der Paratuberkulose von erkrankten Säugern, enthaltend Kunststoffkörper mit einer hydrophoben Oberfläche, an deren Oberfläche eine Schicht eines aus *Mycobacterium paratuberculosis* erhältlichen Lipopolysaccharid- oder Polysaccharid-Antigens eines MG von 30 bis 50 kDa ausgebildet ist, wobei die Testeinrichtung erhältlich ist, indem man ein das Antigen enthaltendes Retentat in eine Pufferlösung einer Salzkonzentration von mindestens 150 mM einbringt und den hydrophoben Kunststoffkörper zur Beschichtung mit dem Antigen mit dem das Retentat enthaltenden Puffer behandelt, und das Retentat erhalten wird durch Behandeln einer *M. paratuberculosis* enthaltenden Bakterien-Suspension mit einer "French-Press", Zentrifugieren, Behandeln des Überstands mit Proteinase K, Zentrifugieren und Filtrieren des Überstands.

Gegenstand der Erfindung ist ferner ein Verfahren zur Herstellung einer immunologischen Testreinrichtung zum Nachweis von Paratuberkulose in dem man ein Retentat, enthaltend ein aus *M. paratuberculosis* erhältliches Lipopolysaccharid- oder Polysaccharid-Antigen mit einem MG von 30 bis 50 kDa in eine Pufferlösung einer Salzkonzentration von mindestens 150 mM einbringt und mit der erhaltenen Lösung hydrophobe Kunststoffplatten oder Kunststoffgefäße behandelt, und das Retentat erhalten wird durch Behandeln einer *M. paratuberculosis* enthaltenden Bakterien-Suspension mit einer _{"}French-Press", Zentrifugieren, Behandeln des Überstands mit Proteinase K, Zentrifugieren und Filtrieren des Überstands.

Gegenstand der Erfindung ist schließlich ein Verfahren zur Erkennung einer Paratuberkulose, in dem man eine Serum- oder Milchprobe eines Säugers in einem geeigneten Puffer in eine immunologische Testeinrichtung, auf deren hydrophober Oberfläche ein aus *M. paratuberculosis* erhältliches Lipopolysaccharid- oder Polysaccharid-Antigen mit einem MG von 30 bis 50 kDa adsorbiert ist, eingebracht, darin inkubiert und nach der ELISA-Methode untersucht wird, wobei zur Adsorption des Antigens auf der hydrophoben Oberfläche diese mit einem das Antigen in einer Pufferlösung einer Salzkonzentration von mindestens 150 mM enthaltenden Retentat behandelt ist, und das Retentat erhalten wird durch Behandeln einer M. *paratuberculosis* enthaltenden Bakterien-Suspension mit einer "French-Press", Zentrifugieren, Behandeln des Überstands mit Proteinase K, Zentrifugieren und Filtrieren des Überstands.

Die Kunststoffkörper können Kunststoffplatten oder Kunststoffgefäße sein, die aus Polystyrol bestehen oder Polystyroloberflächen aufweisen. Sie können derart ausgebildet sein, daß sie für Reihenuntersuchungen nach dem ELISA-Verfahren ausgebildet sind. Andere Kunststoffe kommen auch in Frage, sofern deren Oberfläche hydrophob oder hydrophobiert ist, so daß sich das Antigen daran in ausreichendem Umfang adsorbieren läßt.

Als Antigene zur Beschichtung dieser Oberflächen sind vorzugsweise Lipoarabinomannan- und Arabinomannan-Antigene, die aus *M. paratuberculosis* erfindungsgemäß isoliert werden können, geeignet.

Gemäß einer bevorzugten Ausführungsform der Erfindung setzt sich der Saccharidanteil des Antigens aus etwa 93 Mol-% Glucose, 3 bis 4 Mol-% Mannose, 1 bis 2 Mol-% Arabinose und 0,2 bis 0,8 Mol-% Ribose zusammen und der Proteingehalt ist geringer als 1 %, bezogen auf das Gewicht des Antigens.

Die erfindungsgemäße Testeinrichtung kann dadurch hergestellt werden, daß man das aus *M. paratuberculosis* erhältliche Antigen in einer Pufferlösung einer Salzkonzentration von wenigstens 150 mM löst bzw. darin suspendiert und mit der erhaltenen Lösung oder dem Gemisch die Oberfläche der Kunststoffplatten oder Kunststoffgefäße behandelt, um diese zu beschichten. Es hat sich herausgestellt, daß eine Salzkonzentration im Puffer von 230 bis 300 mM und ein pH-Wert von 5,5 bis 7 besonders vorteilhaft sind. Die zu verwendenden Salze und Puffergemische sind nicht kritisch, es können übliche in der Immunologie häufig verwendete Salze und Puffer eingesetzt werden, wie Natrium- und Kaliumchlorid sowie Phosphatpuffer und Puffer auf der Basis von Citronensäure.

Das zum Beschichten der hydrophoben Oberfläche einzusetzende Antigen wird erhalten durch Kultivieren eines *M. paratuberculosis-Isolats* und Ernten der Kultur, die erhaltene Bakterien-Suspension wird mit einer "French Press" behandelt und zentrifugiert, der Überstand wird einer Behandlung mit Proteinase K unterzogen, zentrifugiert und der Überstand filtriert. Das Retentat wird mit einem Coating-Puffer äquilibriert und zur Beschichtung eingesetzt. Bei dieser Verfahrensweise wird eine mit hohen Verlusten einhergehende Vorreinigung des Antigens vermieden und es steht mehr Antigen zur Beschichtung zur Verfügung. Darüber hinaus wird durch das in Kontaktbringen des Retentats mit dem hydrophoben zu beschichtenden Material und die vergleichsweise selektive Adsorption des Antigens eine weitere Aufreinigung erzielt.

Zur Durchführung des erfindungsgemäßen Verfahrens zur Erkennung der Paratuberkulose werden Serum- oder Milchproben in eine erfindungsgemäße immunologische Testeinrichtung, vorzugsweise in Form einer Mikrotiterplatte, die zur Auswertung durch ein automatisiertes ELISA-Verfahren geeignet ist, eingebracht, darin inkubiert und nach dem ELISA-Verfahren behandelt und ausgewertet.

Erfindungsgemäß wird die immunulogische Testeinrichtung zum Nachweis der Paratuberkulose in Form eines Test-Kit bereitgestellt, wobei das Test-Kit ferner Antikörper gegen das auf Kunststoffplatten oder Kunststoffgefäßen adsorbierten Antigen (positive Referenz), eine negative Referenz und geeignetes Konjugat zur Durchführung eines ELISA enthält. Das erfindungsgemäße diagnostische Verfahren zur Erkennung von *M. paratuberculosis* infizierter Säuger weist einen positiven und einen negativen prädiktiven Wert von jeweils über 85% auf. Für vergleichbare Tests sind prädiktive Werte nicht bestimmt worden.

Die Sensitivität bekannter ELISA wurde mittels nicht repräsentativer, also vorselektierten Stichproben, mit 46% ermittelt. Bei vergleichbarer Vorgehensweise hat das erfindungsgemäße Diagnoseverfahren eine Sensitivität und Spezifität von jeweils über 70%, vorzugsweise über 90%. Dieser Sensitivitäts- und Spezifitätsunterschied würde die Sanierungskosten in einem Bestand entscheidend verbilligen.

Die Spezifität wird ausgedrückt als der Quotient aus der Anzahl der richtig als gesund erkannten Tiere und der Anzahl aller gesunden Tiere. Die Sensitivität wird ausgedrückt als der Quotient aller richtig als krank erkannten Tiere (infizierte als infiziert erkannt) und der Anzahl aller kranken Tiere (infizierte als infiziert erkannt und infizierte als gesund erkannt).

Nachfolgend wird beispielhaft die Präparation des Antigens sowie die Beschichtung von Kunststoffkörpern mit dem Antigen dargestellt.

### Herstellung des M. paratuberculosis Antigens

Das *M. paratuberculosis-Isolat* 6783 wird zunächst auf "Harold's egg yolk Medium" mit Mycobactinzusatz (2 mg/l) angeimpft. Nach 3 Monaten werden 10 ml Flüssigmedium nach Watson-Reid mit Mycobactinzusatz (2 mg/l) in eine 50 ml Gewebekulturflasche (Nunc, Wiesbaden) gefüllt; eine Öse Kulturmaterial vom Schrägagar wird in Pentan resuspendiert und das Flüssigmedium wird überschichtet. Alle 6 bis 8 Wochen wird die Kultur durch Aufteilen in mehrere und größere Flaschen subkultiviert und expandiert; dabei erfolgte jeweils eine Reinheitskontrolle mittels Ziehl-Neelsen-Färbung. Etwa ein Jahr nach Beginn der Kultivierung werden die Kulturen auf ihre Mycobactinabhängigkeit hin überprüft und durch Zentrifugation geerntet (30 min, 2500xg). Das Pellet wird in Aqua dest. resuspendiert (0,6 g Bakterienfeuchtmasse/ml) und hitzeinaktiviert (10 min, 100°C). 10 bis 20 ml der Bakteriensuspension werden einmal mit einer "French Press" behandelt (15.000 psi) und anschließend zentrifugiert (15 min, 6500xg). Das Pellet wird verworfen. Der Überstand wird mit einem gleichgroßen Volumen doppelt konzentriertem Proteinase K-Puffer gemischt (1 x Proteinase K-Puffer ist 10 mM Tris, 5 mM EDTA, 0,5% SDS, ph 7,8) und Proteinase K wird nach Vorverdauung (15 min, 37°C) hinzugefügt (Endkonzentration 1 mg Proteinase K pro g Bakterienfeuchtmasse). Nach Proteinase K Verdau (1 h 37°C) wird das Enzym inaktiviert (5 min, 100°C); es folgt eine Zentrifugation (15 min, 15000xg). und das Pellet wird verworfen. Der Überstand durch ein 0,45 µm Filter filtriert, und es folgt eine Filtration durch ein "Centricon^{R} Zentrifugationfilter". Das Retentat wird einmal mit Coating Puffer (35mM Natriumcitrat äquilibriert auf pH 6 mit 50 mM Citronensäure, 250 mM NaCl) gewaschen. Das gewaschene Retentat wird auf etwa 1 ml pro 2,5 g Bakterienfeuchtmasse eingestellt und in 500 µl Aliquots bei -20°C gelagert. Dieses Retentat enthält etwa 60 mg Trockensubstanz pro ml, und der Proteinanteil liegt unter 1%.

### Beschichten von Kunststoff platten mit dem Antigen

Für den ELISA werden Polysorp^{R}-Platten von NUNC verwendet. Das Beschichten erfolgt bei 37°C 2 Stunden, wobei Antigenkonzentrat (Retentat) und Coatingpuffer vorzuwärmen sind. Das Antigen wird unter Benutzung des positiven Kontrollserums austitriert. Beim Beschichten mit 100 µl ergibt sich eine optimale Coating-Konzentration von 2 µg Trockensubstanz pro ml Coatingpuffer. Die Platten werden viermal mit Waschpuffer (135 mM NaCl, 10 mM Na₂HPO,; 25 mM KCl, 15 mM KH₂PO₄, 0,05% Tween 20, pH 7,6) in einem Plattenwaschgerät (Dynatech AM85) gewaschen und bis zur Benutzung bei -20°C gelagert.

### Das Adsorbieren des Antigens

Zunächst wurde ein Coating-Puffer mit 250 mM NaCl und 35 mM Citronensäure/Citratpuffer, pH 6, hergestellt und darin das bei der Antigenpräperation erhaltene Antigen gelöst. Mit dieser Lösung wurden ELISA-Platten, die unter der Bezeichnung Nunc-Polysorp^{R}-Platten im Handel erhältlich sind, behandelt.

Dieses Verfahren erlaubt das Beschichten von etwa.350 ELISA-Platten mit dem aus 1 g Bakterienfeuchtmasse gewonnenen Antigen. Demgegenüber beschrieben Sugden et al. 1987 das Beschichten von 2,3 ELISA-Platten mit dem aus 1 g Bakterienfeuchtmasse gewonnenen Antigen. Erfindungsgemäß können deshalb nach dem ELISA-Verfahren unter Verwendung von Antigen aus 1 g Bakterienfeuchtmasse wenigstens 3.500 Seren untersucht werden.

Der Einsatz der Polysorp^{R}-Oberfläche ermöglicht ein sehr spezifisches Binden ausschließlich des erfindungsgemaßen Antigens. Durch das Waschen nach der Beschichtungsbehandlung werden Verunreinigungen entfernt, so daß zur Isolierung des Antigens nur das zuvor beschriebene relativ einfache Verfahren durchgeführt werden muß.

## Patentansprüche

1. Immunologische Testeinrichtung zum Nachweis der Paratuberkulose von erkrankten Säugern, enthaltend einen Kunststoffkörper mit einer hydrophoben Oberfläche, an der eine Schicht eines aus *Mycobacterium paratuberculosis* erhältlichen Lipopolysaccharid- oder Polysaccharid-Antigens eines MG von 30 bis 50 kDa ausgebildet ist, wobei die Testeinrichtung erhältlich ist, indem man ein das Antigen enthaltendes Retentat in eine Pufferlösung einer Salzkonzentration von mindestens 150 mM einbringt und den hydrophoben Kunststoffkörper zur Beschichtung mit dem Antigen mit dem das Retentat enthaltenden Puffer behandelt, und das Retentat erhalten wird durch Behandeln einer *M. paratuberculosis* enthaltenden Bakterien-Suspension mit einer "French-Press", Zentrifugieren, Behandeln des Überstands mit Proteinase K, Zentrifugieren und Filtrieren des Überstands.

2. Immunologische Testeinrichtung nach Anspruch 1, **dadurch gekennzeichnet, daß** der Kunststoffkörper eine Kunststoffplatte oder ein Kunststoffgefäß ist und aus Polystyrol besteht oder Polystyroloberflächen aufweist.

3. Immunologische Testeinrichtung nach Anspruch 1 oder 2, **dadurch gekennzeichnet, daß** das Lipopolysaccharid-Antigen ein Lipoarabinomannan ist.

4. Immunologische Testeinrichtung nach Anspruch 1, **dadurch gekennzeichnet, daß** der Saccharidanteil des Antigens etwa 93 Mol-% Glucose, 3 bis 4 Mol-% Mannose, 1 bis 2 Mol-% Arabinose und 0,2 bis 0,8 Mol-% Ribose enthält und der Proteingehalt geringer als 1 %, bezogen auf das Gewicht des Antigens, ist.

5. Immunologische Testeinrichtung nach einem der Ansprüche 1 bis 4 in Form eines Test-Kits zur Durchführung eines ELISA.

6. Verfahren zur Herstellung einer immunologischen Testreinrichtung zum Nachweis von Paratuberkulose nach einem der Ansprüche 1 bis 5, **dadurch gekennzeichnet, daß** man ein Retentat, enthaltend ein aus *M. paratuberculosis* erhältliches Lipopolysaccharid- oder Polysaccharid-Antigen mit einem MG von 30 bis 50 kDa in eine Pufferlösung einer Salzkonzentration von mindestens 150 mM einbringt und mit der erhaltenen Lösung hydrophobe Kunststoffplatten oder Kunststoffgefäße behandelt, und das Retentat erhalten wird durch Behandeln einer *M. paratuberculosis* enthaltenden Bakterien-Suspension mit einer "French-Press", Zentrifugieren, Behandeln des Überstands mit Proteinase K, Zentrifugieren und Filtrieren des Überstands.

7. Immunologisches Verfahren zur Erkennung einer Paratuberkulose, **dadurch gekennzeichnet, dass** eine Serum- oder Milchprobe eines Säugers in einem geeigneten Puffer in eine immunologische Testeinrichtung, auf deren hydrophober Oberfläche ein aus *M. paratuberculosis* erhältliches Lipopolysaccharid- oder Polysaccharid-Antigen mit einem MG von 30 bis 50 kDa adsorbiert ist, eingebracht, darin inkubiert und nach der ELISA-Methode untersucht wird, wobei zur Adsorption des Antigens auf der hydrophoben Oberfläche diese mit einem das Antigen in einer Pufferlösung einer Salzkonzentration von mindestens 150 mM enthaltenden Retentat behandelt ist, und das Retentat erhalten wird durch Behandeln einer *M. paratuberculosis* enthaltenden Bakterien-Suspension mit einer _{"}French-Press", Zentrifugieren, Behandeln des Überstands mit Proteinase K, Zentrifugieren und Filtrieren des Überstands.

## Claims

1. Immunologic test for the detection of paratuberculosis in affected mammals comprising a plastic body with a hydrophobic surface coated with a lipopolysaccharide or polysaccharide antigen with a molecular weight of 30 to 50 kDa prepared from *Mycobacterium paratuberculosis,* whereby test is prepared by adding the retentate containing the antigen in a buffer solution with a minimum salt concentration of 150 mM and using it for coating with the antigen by treating the hydrophobic plastic body with the buffer containing the retentate, and the retentate is acquired by treating a bacterial suspension containing *M. paratuberculosis* with a "French Press", centrifugation, treating the supernatant with Proteinase K, centrifugation and filtration of supernatant.

2. Immunologic test of claim 1, **characterized in that** the plastic body is a plastic plate or a plastic container consisting of poylstyrene or includes polystyrene at its surface.

3. Immunologic test of claim 1 or 2, , **characterized in that** the lipopolysaccharide antigen is a lipoarabinomannan.

4. Immunologic test of claim 1, , **characterized in that** the portion of saccharide of the antigen contains about 93 Mol% glucose, 3 to 4 Mol% mannose, 1 to 2 Mol% arabinose an 0,2 to 0,8 Mol% ribose and protein content is less than 1 % in reference to the weight of the antigen.

5. Immunologic test of one of the claims 1 to 4 in the nature of a test kit for the performance of an ELISA.

6. Procedure for preparation of an immunologic test for the detection of paratuberculosis of claims 1 to 5, , **characterized in that** the a retentate containing a lipopolysaccharide or polysaccharide antigen with a molecular weight of 30 to 50 kDa is added to a buffer solution with a minimum salt concentration of 150 mM and hydrophobic plastic plates or plastic containers are treated with this solution, and retentate is acquired by treating a bacterial suspension containing *M. paratuberculosis* with a "French Press", centrifugation, treating the supernatant with Proteinase K, centrifugation and filtration of supernatant.

7. Immunologic procedure for the recognition of paratuberculosis, , **characterized in that** a serum or milk sample of a mammal in an appropriate buffer is given into an immunologic test, in which hydrophobic surface is absorbed with a lipopolysaccharide or polysaccharide antigen with a molecular weight of 30 to 50 kDa prepared from *Mycobacterium paratuberculosis,* is incubated in it and examined by the ELISA method, whereby for adsorption of the antigen to the hydrophobic surface it is treated with a retentate containing the antigen in a buffer solution with a minimum salt concentration of 150 mM, and retentate is acquired by treating a bacterial suspension containing *M. paratuberculosis* with a "French Press", centrifugation, treating the supernatant with Proteinase K, centrifugation and filtration of supernatant.

## Revendications

1. Test immunologique pour le preuve de Paratuberculose chez de mammifères malades, contenant un object plastique d'une surface hydrophobe qui est sensibilisée d'une couche d'antigène de lipopolysaccharides et de polysaccharides d'un poids moléculaire de 30 à 50 kDa tirée de *Mycobacterium paratuberculosis,* ce teste est obtenu en ingérant un rétentate contenant l'antigène dans un tampon de concentration saline d'au moins 150 mM et en sensibilisant l'object plastique hydrophobe au tampon contenant le rétenate avec l'antigène, le rétentate sera acquis en procédant une suspension bactériologique contenant du *Mycobacterium paratuberculosis* à la « presse francaise », à la centrifugation, au traitement avec de la proteinase K de la fraction liquide, à la centrifugation et la filtration de la fraction liquide.

2. Test immunologique selon la revendication 1, **caractérise en ce que** l'objet plastique est une plaque plastique ou un vase plastique et dont la surface contient polystyrole.

3. Test immunologique selon les revendications 1 et 2, **caractérise en ce que** l'antigène lipopolysaccharide est un lipoarabinomannane.

4. Test immunologique selon la revendication 1, **caractérise en ce que** la fraction saccharifère de l'antigène se constitue de 93 mol% glucose, 3 à 4 mol% mannose, 1 à 2 mol% arabinose et 0,2 à 0,8 mol% ribose et la partie en protides est moins de 1 % relative au poids de l'antigène.

5. Test immunologique selon l'une entre les revendications 1 à 4 en forme d'une trousse pour la réalisation d'un ELISA (anglais = Enzyme-Linked ImmunoSorbend Assay).

6. Le procédé de fabrication d'un test immunologique de reconnaître la Paratuberculose selon l'une entre les revendications 1 à 5, **caractérise en ce que** on ingère un rétentate - contenant un antigène de lipopolysaccharides et de polysaccharides du poids moléculaire de 30 à 50 kDa tiré de M. paratuberculosis - d'un tampon de concentration saline d'au moins 150 mM et dans lequel on traite des plaques plastiques et des vases plastiques à ce solution et dans lequel le rétentate est acquis en procédant une suspension bactériologique contenant du *Mycobacterium paratuberculosis à* la « presse francaise », à la centrifugation, au traitement avec de la proteinase K de la fraction liquide, à la centrifugation et la filtration de la fraction liquide.

7. Un procédé immunologique pour la détection de la Paratuberculose **caractérise en ce que** on met un échantillon de sérum où de lait d'un mammifère ingéré d'un tampon adéquate dans un test immunologique sur sa surface hydrophobe est adsorbé un antigène de lipopolysaccharides et de polysaccharides du poids moléculaire de 30 à 50 kDa tiré de M. paratuberculosis, on incube l'échantillon dedans et l'examine selon la méthode ELISA. A fin d'adsorber l'antigène à la surface hydrophobe on la traite d'un tampon de concentration saline d'au moins 150 mM qui contient le rétenate avec l'antigène et le rétentate est acquis en procédant une suspension bactériologique contenant du *Mycobacterium paratuberculosis* à la « presse francaise », à la centrifugation, au traitement avec de la proteinase K de la fraction liquide, à la centrifugation et la filtration de la fraction liquide.
